# DEMANDE DE BREVET EUROPEEN

(11) **EP 1 632 185 A1**
(43) Date de publication de la demande: **08.03.2006**
(21) Numéro de dépôt: 05291827.3
(22) Date de dépôt: 02.09.2005
(51) Int. Cl.: A61B 17/02

(54) **Rétracteur de tissus permettant l'obtention d'un canal opératoire élargi**

(30) Priorité: 02.09.2004 FR 0409300
(71) Demandeur: Spinevision, 75012 Paris (FR)
(72) Inventeur: Petit, Dominique, 62180 Verton (FR); Sene, Vincent, 94230 Cachan (FR)
(74) Mandataire: Breese Derambure Majerowicz

(57) **Abrégé**

La présente invention se rapporte à un rétracteur (1) de tissus d'un patient, du type comprenant deux lames présentant respectivement une extrémité proximale et une extrémité distale, lesdites lames étant disposées pour former un canal opératoire (6) ouvert aux extrémités proximales et aux extrémités distales desdites lames, caractérisé en ce que le rétracteur (1) comporte au moins une lame complémentaire pour former un rétracteur à trois lames au moins (2 à 5), lesdites lames (2 à 5) s'écartant les unes des autres par pivotement de leurs extrémités distales de sorte à former un canal opératoire (6) élargi de forme conique.

## Description

La présente invention se rapporte au domaine de la rétraction et de l'écartement de tissus mous afin de ménager un champ opératoire permettant d'effectuer des actes chirurgicaux sur un patient.

La présente invention se rapporte plus particulièrement à un rétracteur de tissus d'un patient, du type comprenant des lames présentant respectivement une extrémité proximale et une extrémité distale. Les lames du rétracteur sont disposées pour former un canal opératoire ouvert aux extrémités proximales et distales desdites lames et pouvant être élargi au niveau des extrémités distales desdites lames.

L'art antérieur connaît déjà de tels rétracteurs.

En particulier, il est proposé dans la demande de brevet internationale WO2004/002323 un écarteur comprenant un canal de travail formé par une première partie couplée à une deuxième partie. Lesdites première et deuxième parties peuvent être déplacées l'une par rapport à l'autre d'une configuration non allongée à une configuration allongée, de sorte que les dimensions du canal de travail sont augmentées sur sa longueur, tout en réduisant au minimum le traumatisme causé à la peau et au tissu.

Selon une configuration particulière de l'invention, ces parties pivotent l'une par rapport à l'autre de façon à offrir un canal de travail élargi. Pour ce faire, l'extrémité proximale du rétracteur est munie d'extensions inclinées par rapport au plan horizontal, sur lesquelles sont disposés des membres d'engagement destinés à coopérer avec un instrument de séparation. L'élargissement du canal de travail est alors obtenu par l'application d'une force de séparation latérale sur lesdits membres d'engagement.

Un tel rétracteur présente cependant l'inconvénient de permettre l'écartement du canal de travail seulement dans deux directions de l'espace. Il s'ensuit, pour obtenir l'écartement du canal de travail dans toutes les directions, de disposer dans l'incision, perpendiculairement au rétracteur initialement posé, un second rétracteur ce qui, dans le cas du rétracteur de la demande de brevet internationale susmentionnée, reste relativement difficile à mettre en oeuvre. En outre, le positionnement d'un second rétracteur perpendiculairement au premier conduirait nécessairement à une limitation en surface de la visibilité du canal de travail.

On connaît également de la demande de brevet européen EP0455282 un écarteur chirurgical destiné en particulier à la cholécystectomie.

L'écarteur comprend notamment un cadre écarteur formé par une série de côtés reliés les uns aux autres par des articulations pour former un cadre polygonal articulé, et une pluralité de dilatateurs fixés aux côtés sur le cadre écarteur, lesdits dilatateurs étant d'une forme en plaque substantiellement mince et s'étendant le long d'un axe qui coupe le cadre écarteur.

De par les articulations mises en oeuvre entre chacun des côtés, l'écarteur présente, comme le rétracteur précédemment décrit, l'inconvénient de ne permettre l'écartement des chairs que dans deux directions de l'espace.

La demande de brevet européen EP0246086 porte également sur un rétracteur pour des cholécystectomies laparascopiques.

Le rétracteur comprend notamment un élément de base comportant une ouverture du type fenêtre pour le passage des outils chirurgicaux, et au moins une paire de branches de forme tubulaire.

Or, de par leur configuration, les branches offrent, lorsque écartées, un canal de travail insuffisant pour permettre de pratiquer les actes chirurgicaux en toute sécurité.

En outre, un tel écarteur s'avère de mise en oeuvre peu aisée.

La présente invention entend remédier aux inconvénients de l'art antérieur en proposant un rétracteur pouvant être inséré dans les tissus d'un patient via une incision minimale en surface, tout en permettant un écartement des tissus, en profondeur de l'incision, le plus important possible afin d'offrir un champ opératoire élargi dans les trois directions de l'espace.

La présente invention a également pour but de proposer un rétracteur de mise en oeuvre simple, maniable et non encombrant.

La présente invention a également pour but de proposer un rétracteur permettant un réglage précis de l'amplitude du champ opératoire adaptée aux actes chirurgicaux.

Pour ce faire, la présente invention concerne un rétracteur de tissus d'un patient, du type comprenant deux lames présentant respectivement une extrémité proximale et une extrémité distale, lesdites lames étant disposées pour former un canal opératoire ouvert aux extrémités proximales et aux extrémités distales desdites lames, et elle est remarquable en ce que le rétracteur comporte au moins une lame complémentaire pour former un rétracteur à trois lames au moins, lesdites lames s'écartant les unes des autres par pivotement de leurs extrémités distales de sorte à former un canal opératoire élargi de forme conique.

Ainsi, le rétracteur selon l'invention permet d'écarter les tissus dans les trois directions de l'espace, contrairement aux rétracteurs de l'art antérieur lesquels permettent une distraction des tissus seulement dans deux directions.

Selon un mode de réalisation préféré de l'invention, le rétracteur comporte un dispositif d'écartement des lames, constitué par au moins deux éléments d'appui permettant d'exercer une force sur l'extrémité proximale desdites lames, et ainsi générer leur écartement.

Avantageusement, les éléments d'appui constituent des éléments de réglage de l'amplitude d'écartement desdites lames.

Avantageusement, les éléments d'appui consistent en des vis, chacune desdites vis traversant un orifice fileté formé dans lesdites lames.

Selon un mode de réalisation avantageux de l'invention, le rétracteur comporte en outre une membrane de protection tubulaire entourant lesdites branches.

Avantageusement, les lames présentent des encoches de maintien pour maintenir fixe la membrane autour desdites lames.

Avantageusement, la membrane est élastique.

Avantageusement, la membrane est tissulaire ou en polymère, du type polyuréthane.

Avantageusement, les lames sont amovibles.

Avantageusement, les lames sont constituées de matériau radiotransparent.

Avantageusement, le rétracteur comporte au moins un orifice d'accès permettant un accès à l'espace situé entre les tissus et les lames ou, lorsque le rétracteur comporte une membrane de protection telle que décrite précédemment, un accès à l'espace situé entre la membrane et les lames entourées de ladite membrane. Le rétracteur, ainsi muni d'un tel orifice, permet le passage d'un outil, tel qu'un outil d'aspiration ou un outil délivrant de la lumière froide. L'outil, alors disposé entre les tissus et les lames ou, lorsque le rétracteur comprend une membrane, entre ladite membrane et les lames, n'obture pas le canal de travail.

Avantageusement, le rétracteur présente une partie proximale de forme évasée pour permettre une bonne visibilité du canal de travail.

Avantageusement, le rétracteur comporte des moyens de fixation sur un support, du type table, permettant ainsi de stabiliser le rétracteur une fois disposé dans l'incision formée dans les tissus du patient.

La présente invention se rapporte également à un rétracteur de tissus d'un patient, du type comprenant deux lames présentant respectivement une extrémité proximale et une extrémité distale, lesdites lames étant disposées pour former un canal opératoire ouvert aux extrémités proximales et aux extrémités distales desdites lames, caractérisé en ce que le rétracteur comporte une membrane de protection tubulaire entourant lesdites lames.

Une telle membrane permet d'éviter que les tissus écartés ne pénètrent dans les espaces formés entre les branches lorsque celles-ci sont en position écartée, de sorte que le canal opératoire offre un champ opératoire optimal.

De préférence, le rétracteur comporte au moins une lame complémentaire pour former un rétracteur à trois lames au moins, lesdites lames étant entourées par la membrane.

Avantageusement, les lames présentent des encoches de maintien pour maintenir fixe la membrane autour desdites lames.

Avantageusement, la membrane est élastique.

Avantageusement, la membrane est tissulaire ou en polymère, et de préférence en polyuréthane.

Avantageusement, ledit rétracteur comprendra également les caractéristiques techniques du rétracteur précédemment décrit (dispositif d'écartement, lames amovibles, lames constituées de matériau radiotransparent, orifice d'accès, forme proximale évasée, moyens de fixation sur un support).

On comprendra mieux l'invention à l'aide de la description, faite ci-après à titre purement explicatif, d'un mode de réalisation de l'invention, en référence aux figures annexées où :
- la figure 1 illustre une vue en perspective d'un rétracteur de tissus selon l'invention lorsque le rétracteur est en position écartée ;
- la figure 2 illustre une vue en perspective du rétracteur de la figure 1 en position rétractée ;
- les figures 3 et 4 illustrent le rétracteur des figures 1 et 2 selon des positions intermédiaires d'écartement ;
- la figure 5 illustre une vue en perspective du rétracteur de la figure 1 muni d'une membrane de protection, en position rétractée ; et
- la figure 6 illustre une vue en perspective du rétracteur de la figure 5 en position écartée.

Comme cela a été précédemment expliqué, un rétracteur de tissus est un appareil permettant d'effectuer des actes chirurgicaux sur un patient.

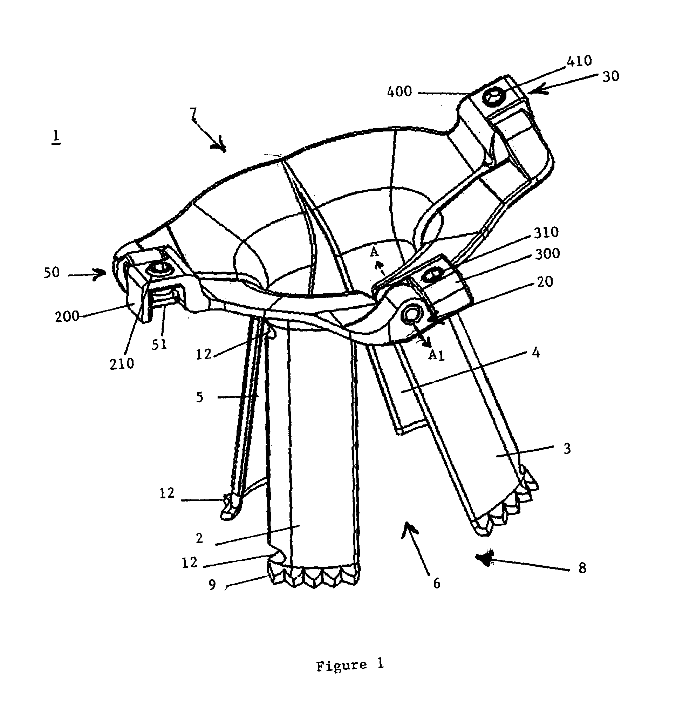

La figure 1 illustre un rétracteur (1) de tissus selon l'invention lorsque celui-ci est dans une position écartée.

Dans l'exemple illustré, le rétracteur (1) comporte quatre lames (2 à 5), chacune des lames (2 à 5) présentant respectivement une extrémité proximale et une extrémité distale.

Lesdites lames (2 à 5) sont disposées les unes par rapport aux autres de manière à former un canal opératoire (6) ouvert à ses extrémités, les extrémités proximales et les extrémités distales de chacune desdites lames (2 à 5) constituant respectivement l'extrémité proximale (7) et l'extrémité distale (8) dudit rétracteur (1).

Chacune des lames (2 à 5) est raccordée avec au moins une lame adjacente au niveau de leur extrémité proximale respectivement au moyen d'une liaison pivot (20, 30, 50) présentant un axe de pivotement AA1 perpendiculaire à l'axe longitudinal BB1 du rétracteur (1). Par axe longitudinal, on entend l'axe longitudinal du rétracteur (1) lorsque ce dernier est en position rétractée.

Ainsi, la lame (5) est raccordée à la lame (2) par la liaison pivot (50), la lame (2) à la lame (3) par la liaison pivot (20), et la lame (3) à la lame (4) par la liaison (40). En revanche, il n'est pas procédé à une fixation des lames (4, 5), celles-ci étant déjà fixées respectivement aux lames (3, 2).

L'extrémité proximale (7) dudit rétracteur (1) est munie de trois éléments de retenue (200, 300, 400) destinés à recevoir respectivement une extension des liaisons pivot (20, 30 50), chaque extension venant en butée contre un desdits éléments de retenue (200, 300, 400).

En outre, lesdits éléments de retenue (200, 300, 400) permettent de bloquer tout mouvement de translation par rapport à l'axe de pivotement AA1 des liaisons pivots (20, 30, 50), et ainsi éviter un éventuel démontage des lames du rétracteur.

Avantageusement, les éléments de retenue (200, 300, 400), en forme de U inversé, sont disposés sur l'extrémité proximale de la lame adjacente à la lame portant la liaison pivot, l'extension de la liaison pivot s'étendant en direction dudit élément de retenue correspondant. Ainsi, les éléments de retenue (200, 300, 400) sont portés sur les extrémités proximales des lames (20, 30, 40).

Chaque lame (2 à 5) du rétracteur (1) ainsi raccordée peut être écartée indépendamment des lames adjacentes par un mouvement de pivotement en direction des tissus, puis ramenée jusqu'à la position rétractée « autorisée ».

L'écartement des quatre lames (2 à 5) du rétracteur (1) est obtenu par l'application d'une poussée sur l'extension de chacune des liaisons pivot (20, 30, 50). Avantageusement, la pression exercée est appliquée au moyen d'un élément d'appui (210, 310, 410), du type vis, laquelle traverse un orifice fileté de l'élément de retenue correspondant pour être en contact avec l'extension de la liaison pivot associée.

Ainsi, lorsque les vis d'appui (210, 310, 410) sont vissées dans les éléments de retenue (200, 300, 400) comme illustré sur la figure 1, l'extension des liaisons pivot est poussée vers le bas, entraînant le mouvement de pivotement des lames portant la liaison pivot correspondante.

Chaque vis d'appui actionne donc l'écartement de deux lames adjacentes. Ainsi, l'écartement des lames (2, 5) est obtenu en agissant sur la vis d'appui (210), l'écartement des lames (2, 3) en agissant sur la vis d'appui (310), l'écartement des lames (3, 4) en agissant sur la vis d'appui (410).

Il est bien entendu évident que le dispositif de vis décrit ci-dessus est donné ici à titre exemple, et que tout autre moyen permettant l'écartement des lames les unes des autres pourra être utilisé sans sortir du champ de la présente invention.

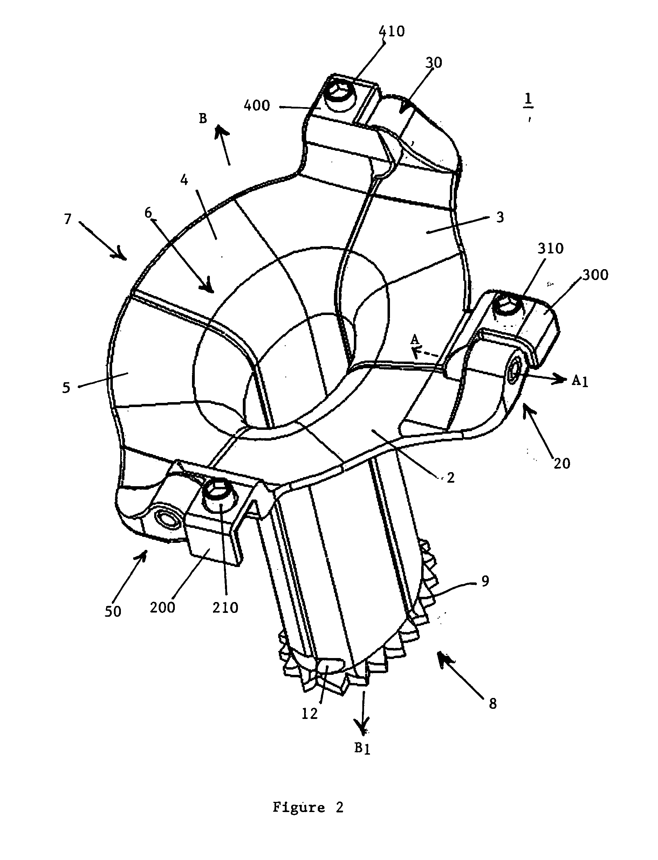

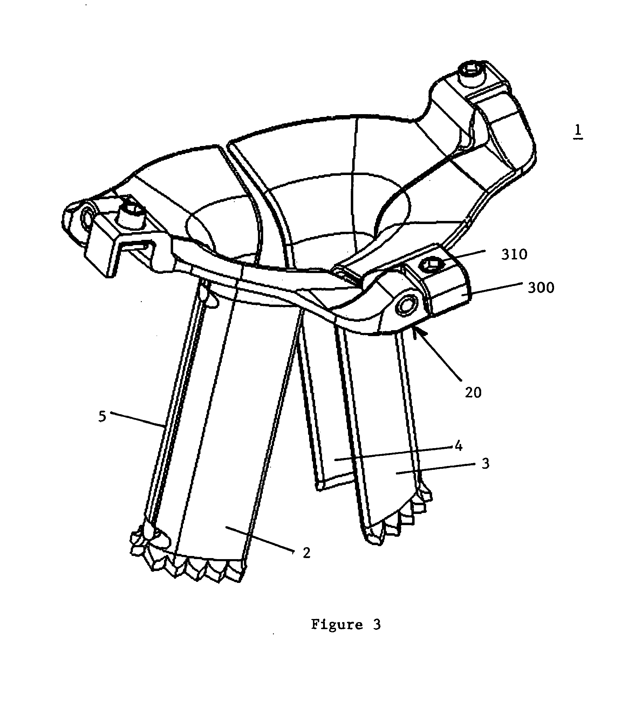

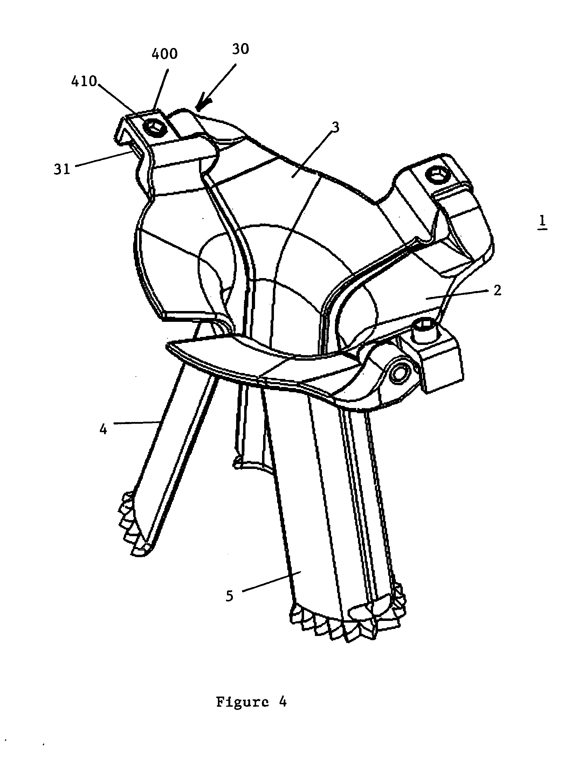

Les figures 3 et 4 illustrent les positions successives qui sont conférées aux lames (2 à 5) du rétracteur (1) pour passer de la position rétractée de la figure 2 à la position écartée de la figure 1 du rétracteur (1).

En particulier, la figure 3 illustre l'écartement de la paire de lames (2, 5) avec la paire de lame (3, 4) du rétracteur (1) obtenu lorsque la vis d'appui (310) est actionnée en premier lieu.

En effet, lorsque la vis d'appui (310) est vissée dans l'élément de retenue (300) correspondant, ladite vis d'appui (310) exerce une poussée sur l'extension de la liaison pivot (20), entraînant ainsi le pivotement de la lame (2) par rapport à la lame (3). La lame (5) n'étant pas physiquement fixées à la lame (4), le pivotement de la lame (2) par rapport à la lame (3) entraîne le pivotement de la lame (5) par rapport à la lame (4), les lames (5, 4) étant respectivement fixées aux lames (2, 3).

La figure 4 illustre, l'écartement de deux autres lames, lorsqu'il est procédé au vissage successif d'une nouvelle vis d'appui. En l'occurrence, la vis d'appui illustrée sur la figure 4 est la vis référencée 410. En vissant la vis d'appui (410) dans l'élément de retenue (400) correspondant, ladite vis (410) exerce une poussée sur l'extension (31) de la liaison pivot (30), entraînant ainsi le pivotement de la lame (4) par rapport à la lame (3).

Afin d'obtenir la position écartée optimale du rétracteur (1) illustrée sur la figure 1, il suffit alors de visser la vis d'appui (210) restante dans l'élément de retenue (200) correspondant, ladite vis d'appui (210) exerçant une poussée sur l'extension (51) de la liaison pivot (50) de sorte à entraîner le pivotement de la lame (5) par rapport à la lame (2).

Il est bien entendu évident que la présente invention ne se limite pas à l'ordre de vissage précédemment décrit.

Il convient en outre de noter que le dispositif d'écartement décrit a pour avantage non seulement de maintenir fermement les lames du rétracteur (1) dans une position écartée, mais également de régler l'amplitude de l'écartement des lames (2 à 5) dudit rétracteur (1).

Par ailleurs, de la description qui précède, il apparaît clairement que les lames (2 à 5) du rétracteur (1) peuvent être écartées les unes des autres de manière indépendante.

Avantageusement, lesdites lames (2 à 5) sont en forme de tuile de sorte que lorsque le rétracteur (1) est en position rétractée, en particulier lors de son insertion dans les tissus du patient, le canal opératoire (6) formé par lesdites lames (2 à 5) est de forme ovale (cf. figure 2).

Avantageusement, les lames (2 à 5) présentent une extrémité proximale configurée de telle sorte que, lorsqu'elles sont raccordées les unes aux autres, le rétracteur (1) présente une partie proximale (7) de forme évasée, ayant l'avantage d'offrir un bon champ de vision du canal opératoire (6).

Les lames (2 à 5) ainsi disposées et configurées, le rétracteur (1), en position rétractée, présente sensiblement une forme d'entonnoir, les parois de cet entonnoir étant constituées par lesdites lames (2 à 5).

Avantageusement, lesdites lames (2 à 5) présentent sur leurs extrémités distales des dents (9) s'étendant transversalement en direction des tissus.

Avantageusement, lesdites lames (2 à 5) sont en inox, aluminium, titane ou polymère.

Dans un mode de réalisation préférée, lesdites lames (2 à 5) seront constituées de matériau radiotransparent.

De même, selon un mode de réalisation avantageux de l'invention, lesdites lames (2 à 5) sont amovibles par rapport au dispositif d'écartement. Ainsi, à tout moment, il est possible de retirer une lame, par exemple trop courte, et de la remplacer par une nouvelle lame plus longue, ou inversement. De même, il sera possible de remplacer chacune des lames sans qu'il soit nécessaire, alors que l'écarteur est disposé dans l'incision, de retirer le dispositif d'écartement.

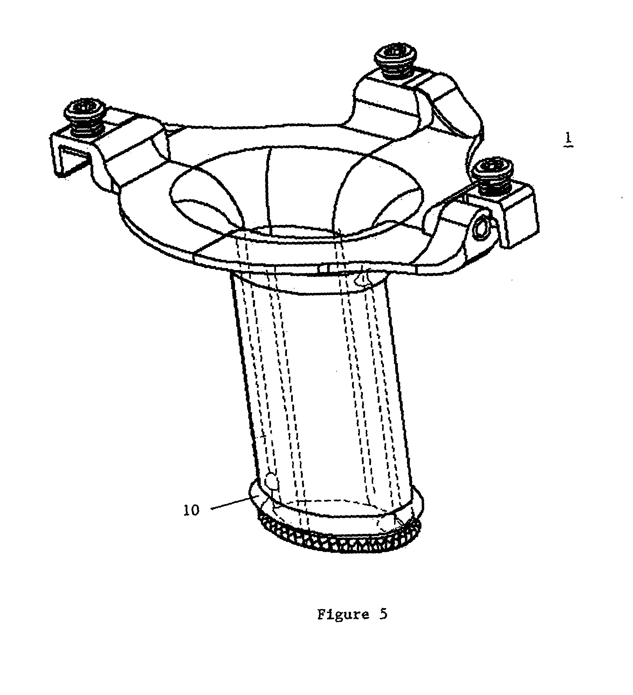

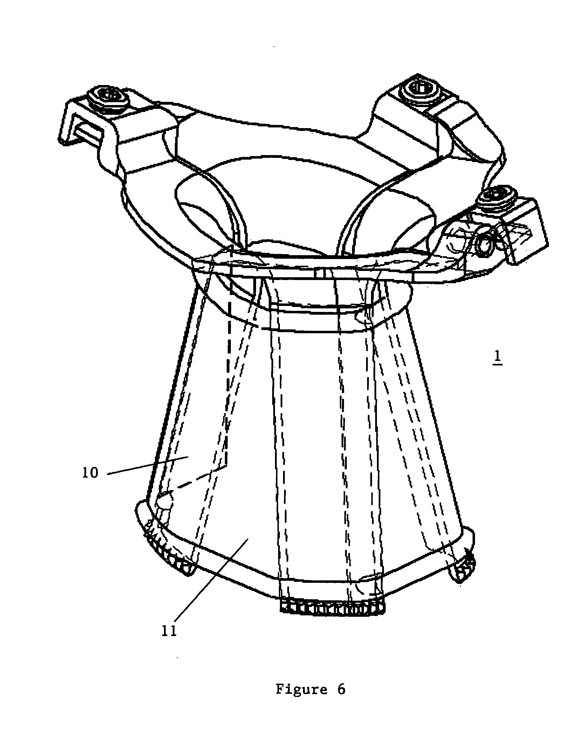

Les figures 5 et 6 illustrent le rétracteur (1) muni d'une membrane (10) de protection tubulaire.

Ladite membrane (10), avantageusement élastique, entoure les lames du rétracteur (1). Elle est maintenue sur les lames au moyen d'encoches (12) formées sur les lames (2 à 5) (cf. figure 2).

Ainsi, lorsque le rétracteur est en position écartée, la membrane permet d'éviter que des tissus écartés ne pénètrent dans les espaces (11) formés entre les lames (2 à 5). Ainsi, le rétracteur (1) équipé de la membrane (10) présente un canal opératoire vierge de tout tissu. En outre, la membrane (10) permet de limiter dans le canal opératoire (6) le saignement. Le saignement ainsi réduit offre un meilleur champ de vision du canal opératoire (6), impliquant un bénéfice immédiat pour le patient.

Avantageusement, la membrane est tissulaire ou en polymère du type polyuréthane.

Selon un mode de réalisation avantageux de l'invention (non représenté), la membrane (10) est fixée non déployée autour des lames (2 à 5) du rétracteur (1) lorsque celui-ci est en position rétractée et se déploie le long des lames (2 à 5) simultanément avec leur écartement.

L'invention est décrite dans ce qui précède à titre d'exemple. Il est entendu que l'homme du métier est à même de réaliser différentes variantes de l'invention sans pour autant sortir du cadre du brevet.

## Revendications

1. Rétracteur (1) de tissus d'un patient, du type comprenant deux lames présentant respectivement une extrémité proximale et une extrémité distale, lesdites lames étant disposées pour former un canal opératoire (6) ouvert aux extrémités proximales et aux extrémités distales desdites lames, **caractérisé en ce que** le rétracteur (1) comporte au moins une lame complémentaire pour former un rétracteur (1) à trois lames au moins (2 à 5), lesdites lames (2 à 5) s'écartant les unes des autres par pivotement de leurs extrémités distales de sorte à former un canal opératoire (6) élargi de forme conique.

2. Rétracteur (1) de tissus d'un patient selon la revendication 1, **caractérisé en ce que** le rétracteur (1) comporte un dispositif d'écartement des lames (2 à 5) constitué par au moins deux éléments d'appui permettant d'exercer une force sur l'extrémité proximale desdites lames (2 à 5) générant l'écartement desdites lames (2 à 5).

3. Rétracteur (1) de tissus d'un patient selon la revendication précédente, **caractérisé en ce que** les éléments d'appui constituent des éléments de réglage de l'amplitude d'écartement desdites lames (2 à 5).

4. Rétracteur (1) de tissus d'un patient selon la revendication 2 ou la revendication 3, **caractérisé en ce que** les éléments d'appui consistent en des vis (210, 310, 410), chacune desdites vis (210, 310, 410) traversant un orifice fileté formé dans lesdites lames.

5. Rétracteur (1) de tissus d'un patient selon l'une quelconque des revendications 1 à 4, **caractérisé en ce que** le rétracteur (1) comporte en outre une membrane de protection (10) tubulaire entourant lesdites lames.

6. Rétracteur (1) de tissus d'un patient selon la revendication précédente, **caractérisé en ce que** les lames présentent des encoches de maintien (12) pour maintenir fixe la membrane (10) autour desdites lames.

7. Rétracteur (1) de tissus d'un patient selon la revendication 5 ou la revendication 6, **caractérisé en ce que** la membrane (10) est élastique.

8. Rétracteur (1) de tissus d'un patient selon l'une quelconque des revendications 5 à 7, **caractérisé en ce que** la membrane (10) est tissulaire ou en polymère.

9. Rétracteur (1) de tissus d'un patient selon la revendication précédente, **caractérisé en ce que** la membrane (10) est en polyuréthane.

10. Rétracteur (1) de tissus d'un patient selon l'une quelconque des revendications 1 à 9, **caractérisé en ce que** les lames (2 à 5) sont amovibles.

11. Rétracteur (1) de tissus d'un patient selon l'une quelconque des revendications 1 à 10, **caractérisé en ce que** les lames (2 à 5) sont constituées de matériau radiotransparent.

12. Rétracteur (1) de tissus d'un patient selon l'une quelconque des revendications 1 à 11, **caractérisé en ce que** le rétracteur (1) comporte au moins un orifice d'accès permettant un accès à l'espace situé entre les tissus et les lames ou, lorsque le rétracteur comporte une membrane de protection conformément aux revendications 5 à 9, un accès à l'espace situé entre la membrane et les lames entourées de ladite membrane.

13. Rétracteur (1) de tissus d'un patient selon l'une quelconque des revendications 1 à 12, **caractérisé en ce que** le rétracteur (1) présente une partie proximale (7) de forme évasée.

14. Rétracteur (1) de tissus d'un patient selon l'une quelconque des revendications 1 à 13, **caractérisé en ce que** le rétracteur (1) comporte des moyens de fixation sur un support.
